# EUROPEAN PATENT SPECIFICATION

(11) **EP 1 596 772 B1**
(45) Date of publication and mention of the grant of the patent: **13.04.2011**
(21) Application number: 04710664.6
(22) Date of filing: 12.02.2004
(51) Int. Cl.: A61F 2/44

(54) **DEVICE FOR FUSING TWO BONE SEGMENTS**
VORRICHTUNG ZUR FUSION ZWEIER KNOCHENSEGMENTE
DISPOSITIF DE FUSION DE DEUX SEGMENTS OSSEUX

(30) Priority: 12.02.2003 US 446963 P; 06.05.2003 US 430473
(43) Date of publication of application: 23.11.2005
(73) Proprietor: Warsaw Orthopedic, Inc., Warsaw, IN 46581 (US)
(72) Inventor: EISERMANN, Lukas, Memphis, TN 38103 (US)
(74) Representative: Kühn, Armin
(86) International application number: PCT/US2004/004109
(87) International publication number: WO 2004/071346

(56) References cited:
- WO-A-01/93785
- WO-A-02/30337
- FR-A- 2 742 653
- US-A- 5 591 235

## Description

### Background

The present disclosure relates generally to the field of orthopedics and spinal surgery, and in some embodiments, the present disclosure relates to fusion-promoting prosthetic devices for insertion into an intervertebral disc space.

In the treatment of diseases, injuries or malformations affecting spinal motion segments, and especially those affecting disc tissue, it has long been known to remove some or all of a degenerated, ruptured or otherwise failing disc. In cases involving intervertebral disc tissue that has been removed or is otherwise absent from a spinal motion segment, corrective measures are taken to ensure the proper spacing of the vertebrae formerly separated by the removed disc tissue. In some instances, fusion-promoting prosthetic devices, such as fusion cages and the like, are inserted into the disc space to maintain the structural integrity of the spinal column.

Anterior plating is often used in conjunction with fusion devices to supplement the stability provided by such fusion devices. However, in some instances, anterior plating is inappropriate for use due to the presence of vascular structure, which impedes the implantation and positioning of the anterior plating.

FR 2 742 653 A discloses an intervertebral cage comprising a circumferential wall defining a through hole divided into three compartments by two separation walls which extend beyond the upper and lower surfaces of the cage for engaging into the adjacent vertebrae when the cage is in the implanted condition.

Therefore, what is needed is an implantable fusion device which eliminates, or at least reduces, the need for supplemental plating external of the intervertebral space.

### Summary

The invention provides a fusion-promoting prosthetic device for insertion into an intervertebral space according to claim 1. Further embodiments of the invention are described in the dependent claims. The prosthetic device includes a sagitally-extending plate having caudal and cephalad edges, the caudal edge being adapted for complete insertion within a first vertebral body and the cephalad edge being adapted for complete insertion within a second vertebral body adjacent to the first vertebral body. The prosthetic device further includes a first transverse plate connected to the sagitally-extending plate, and a second transverse plate connected to the sagitally-extending plate, the first and second transverse plates being adapted for complete insertion within the intervertebral space.

### Brief Description of the Drawings

Fig. 1 is a lateral view of a pair of adjacent vertebral bodies.
Fig. 2 is a lateral view of a prosthetic device for insertion between the adjacent vertebral bodies of Fig. 1.
Fig. 3a is a longitudinal view of the prosthetic device of Fig. 2.
Fig. 3b is a longitudinal view of an alternative prosthetic device.
Fig. 4a is a longitudinal view of a pair of the verterbral bodies of Fig. 1 having longitudinally-formed slots for receiving the prosthetic device of Fig. 2.
Fig. 4b is a lateral view of the prosthetic device of Fig. 2 shown longitudinally disposed between the vertebral endplates of Fig. 1.
Fig. 5a is a lateral view of a pair of verterbral bodies having laterally-formed slots for receiving a prosthetic device.
Fig. 5b is a lateral view of an alternative prosthetic device shown laterally disposed between the vertebral bodies of Fig. 5a.

### Description

For the purposes of promoting an understanding of the principles of the disclosure, reference will now be made to the embodiments, or examples, illustrated in the drawings and specific language will be used to describe the same, by way of example only.

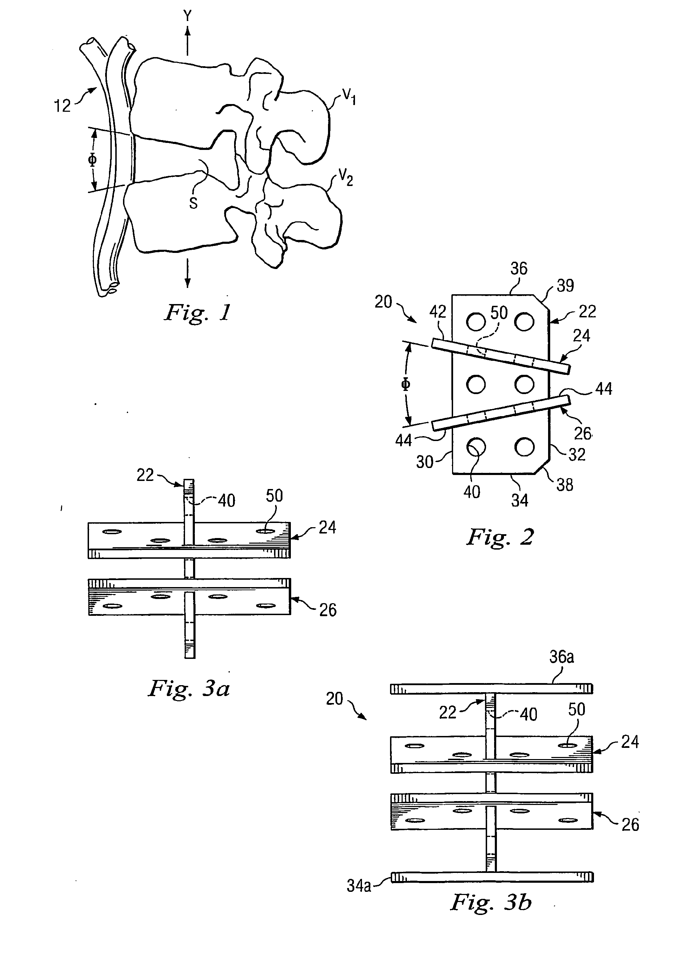

Referring now to Fig. 1, shown therein is a lateral view of a portion of a spinal column 10, illustrating a pair of adjacent upper and lower vertebrae V1 and V2, respectively, separated by an intervertebral space S created by the removal of a natural intervertebral disc. The illustration of two vertebrae is only intended as an example. Another example would be a sacrum and one vertebrae. Vascular structure 12, such as, for example, the aortic artery and associated segmental arteries, is shown disposed anteriorly adjacent to the upper and lower vertebrae V1, V2. As can be appreciated, it is not desirable to impart pressure to, or otherwise contact, the vascular structure 12 during insertion and upon implantation of a prosthetic device.

Referring now to Figs. 2 and 3a, a prosthetic device for insertion into the space S (Fig. 1) is generally referred to by reference numeral 20. In one embodiment, the device 20 includes a sagittally-extending support plate 22 and a pair of additional support plates 24, 26 integrally formed with and extending generally transverse to the sagittal plate. It is understood that the transverse plates 24, 26 may alternatively be removably connected to the sagittal plate 22 such that the prosthetic device 20 is generally modular in nature. Although the sagittal plate 22 and the transverse plates 24, 26 of the prosthetic device 20 may be formed from a wide variety of materials, in one embodiment of the disclosure, the sagittal plate 22 and the transverse plates 24, 26 are formed of a cobalt-chrome-molybdenum metallic alloy (ASTM F-799 or F-75). However, in alternative embodiments of the disclosure, the sagittal plate 22 and the transverse plates 24, 26 may be formed of other materials such as titanium or stainless steel, a polymeric material such as polyethylene, or any other biocompatible material that would be apparent to one of ordinary skill in the art.

The sagittal plate 22 is adapted to engage the upper and lower vertebrae V1, V2, respectively, and in the present example, the sagittal plate 22 extends in a plane substantially parallel to the sagittal plane (represented by axis Y lying in the sagittal plane in Fig. 1) when engaged with the upper and lower vertebrae. The sagittal plate 22 includes an anterior edge 30, a posterior edge 32, a caudal edge 34 and a cephalad edge 36. It is understood that reference to anatomical directions in this specification such as sagittal, anterior, posterior, caudal and cephalad is for purposes of descriptive clarity only, and is not intended to limit the prosthetic device 20 to having a specific orientation relative to such anatomical directions.

The caudal and cephalad edges 34, 36 of the sagittal plate 22 are formed as keel-like structures, which aid in the insertion of the prosthetic device 20 into the intervertebral space S. For example, in one embodiment, the caudal edge 34 of the sagittal plate 22 is beveled at the posterior portion thereof to provide a sharp, forward edge 38 with which to pierce the vertebral body V2 upon insertion of the prosthetic device 20. In a like manner, the cephalad edge 36 of the sagittal plate 22 is beveled at the posterior portion thereof to provide a sharp, forward edge 39 with which to pierce the vertebral body V1 upon insertion of the prosthetic device 20.

It should be understood that other shapes and orientations of the caudal and cephalad edges 34, 36 are also contemplated. For example, the caudal and cephalad edges 34, 36 may be angled along the entire surface thereof to aid in the circumvention of vascular structure 12, or other obstacles, that may be in place during insertion of the prosthetic device 20. Also, the caudal and cephalad edges 34, 36 may be angled, tapered, or configured in some other shape to facilitate the functional demands of insertion. In still another embodiment, such as the one depicted in Fig. 3b, the caudal and cephalad edges 34, 36 may be configured as having winged portions, including a transverse portion 34a, 36a extending across each edge 34, 36, respectively.

Referring again to Figs. 2 and 3a, the sagittal plate 22 is adapted to promote fusion between the vertebral bodies V1, V2 (Fig. 1), and as such, in one embodiment, a plurality of openings 40 are defined through the sagittal plate to promote fusion therethrough. It should be understood that any number of openings 40 may be defined through the sagittal plate 22, including a single opening or two or more openings. It should also be understood that the openings 40 need not necessarily extend entirely through the sagittal plate 22, but may alternatively extend partially therethrough. It should further be understood that the sagittal plate 22 need not necessarily define any openings 40 extending either partially or entirely therethrough. Additionally, although the openings 40 are illustrated as having a circular configuration, it should be understood that other sizes and configurations of the openings 40 are also contemplated.

To further promote fusion, the sagittal plate 22 is preferably coated with a bone-growth promoting substance, such as, for example, a hydroxyapatite coating formed of calcium phosphate. Additionally, the sagittal plate 22 may be roughened prior to being coated with the bone-growth promoting substance to further enhance bone on-growth. Such surface roughening may be accomplished by way of, for example, acid etching, knurling, application of a bead coating, or other methods of roughening that would occur to one of ordinary skill in the art.

The sagittal plate 22 may include one or more notches (not shown) or other types of indicia for receiving or engaging with a corresponding portion of a surgical instrument (not shown) to aid in the manipulation and insertion of the prosthetic device 20 within the intervertebral space S (Fig. 1) between the adjacent vertebral bodies V1, V2 (Fig. 1). The surgical instrument (not shown) is preferably configured to release the sagittal plate 22 once properly positioned between the adjacent vertebrae. One example of a surgical instrument that can be used to insert the prosthetic device 20 is described in co-pending application U.S. Serial No. 10/430,473.

The transverse plates 24, 26 are adapted to engage the vertebral bodies V1, V2 via a pair of bearing surfaces 42, 44, respectively. In the present example, the transverse plates 24, 26 angle towards one another in the posterior direction to accommodate an angular relationship Φ defined between the upper and lower vertebrae V1, V2. As can be appreciated, the angular relationship between the vertebral bodies V1, V2 will vary depending on the particular region of the spine such as the thoracic and lumbar regions. Moreover, a variety of conditions can contribute to a variety of more pronounced angular relationships between the vertebral bodies V1, V2, such as lordosis, kyphosis, etc., and therefore, the transverse plates 24, 26 may extend across the sagittal plate 22 at a variety of angles relative to the sagittal plate, including at right angles.

As with the sagittal plate 22, the transverse plates 24, 26 are adapted to promote fusion between the vertebral bodies V1, V2, and as such, a plurality of openings 50 are defined through each of the transverse plates to promote fusion therethrough. It should be understood that any number of openings 50 may be defined through the transverse plates 24, 26, including a single opening or two or more openings. It should also be understood that the openings 50 need not necessarily extend entirely through the transverse plates 24, 26, but may alternatively extend partially therethrough. It should further be understood that the transverse plates 24, 26 need not necessarily defme any openings 50 extending either partially or entirely therethrough. Additionally, although the openings 50 are illustrated as having a circular configuration, it should be understood that other sizes and configurations of the openings 50 are also contemplated.

To further promote fusion, the transverse plates 24, 26 are preferably coated with a bone-growth promoting substance, such as, for example, a hydroxyapatite coating formed of calcium phosphate. Additionally, the transverse plates 24, 26 may be roughened prior to being coated with the bone-growth promoting substance to further enhance bone on-growth. Such surface roughening may be accomplished by way of, for example, acid etching, knurling, application of a bead coating, or other methods of roughening that would occur to one of ordinary skill in the art.

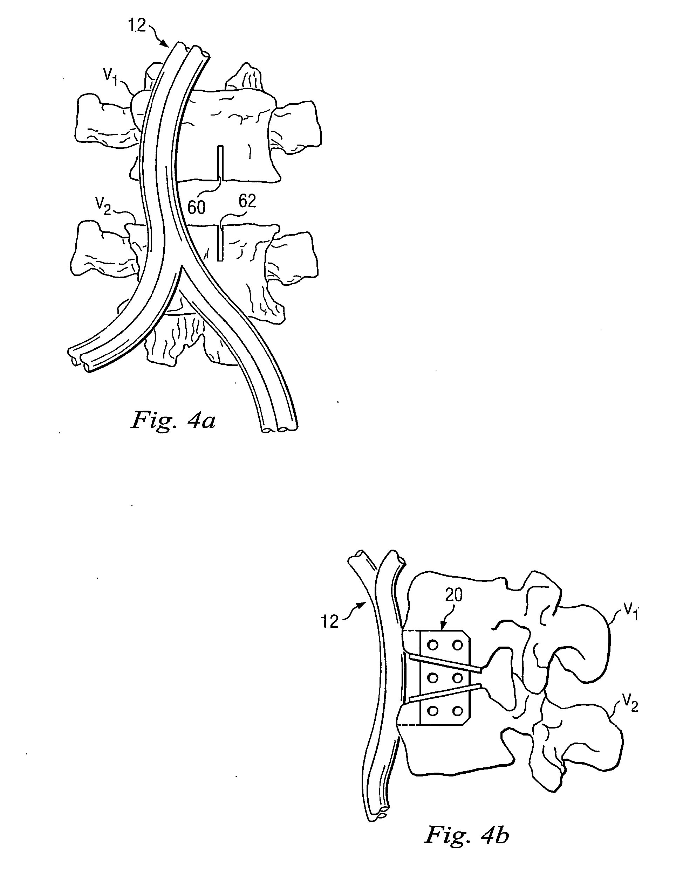

Referring to Figs. 4a and 4b, the prosthetic device 20 (Figs. 2 and 3) may be inserted into the space S between the vertebrae V1, V2 from a variety of approaches. For example, in operation, to accommodate insertion of the prosthetic device 20, the vertebral bodies V1, V2 can be prepared to accept the prosthetic device therebetween from an offset longitudinal, or anterior-oblique, approach. Specifically, elongate openings or slots 60, 62 may be formed in the vertebral endplates of the upper and lower vertebrae V1, V2, respectively, at a predetermined width and to a predetermined depth. The slots 60, 62 can be substantially aligned with each other to accommodate the sagittal plate 22, and more specifically, to accommodate the caudal and cephalad edges 34, 36 defined on the sagittal plate. In one embodiment, the elongate slots 60, 62 are rectangular-shaped and are formed by chiseling or curetting. However, other methods of forming the slots 60, 62 are also contemplated as would occur to one of ordinary skill in the art, such as, for example, by drilling or reaming. Furthermore, for some embodiments of the prosthetic device 20, the caudal and cephalad edges 34, 36 can form their own corresponding slots by engagement and impaction of the beveled edges 38, 39 with the vertebrae V1, V2, and thus no preformed slots are necessary.

As is readily apparent from Fig. 4b, upon insertion into the intervertebral space S defined between the vertebrae V1, V2, the prosthetic device 20 is completely disposed within the intervertebral space S such that no portion of the prosthetic device extends beyond the anterior or posterior portion of the vertebrae V1, V2. Moreover, no plating external of the intervertebral space S is required upon insertion of the prosthetic device 20, which is advantageous in avoiding the problems associated with contacting the vascular structure 12. In addition, the disposition of the prosthetic device 20 in the intervertebral space S results in a relatively large graft area between the vertebrae V1, V2, the graft area being defined, in one embodiment, by that portion of the intervertebral space S not occupied by the prosthetic device 20. As such, bone grafts (not shown) may be inserted into the graft area between the vertebrae V1, V2 to encourage fusion between the vertebrae. Moreover, although not required, supplemental screws (not shown) may be impacted into the vertebrae V1, V2 to provide additional support. Such screws, however, can be press-fit completely into the vertebrae V1, V2 such that no portion of the screws extends beyond the anterior or posterior portion of the vertebrae V1, V2.

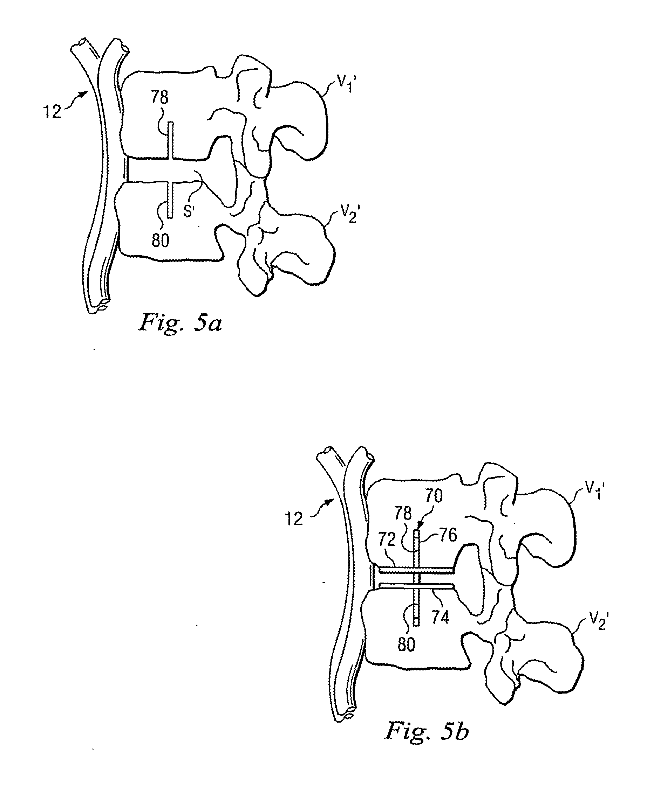

Referring now to Figs. 5a and 5b, in another embodiment, a prosthetic device 70 may be laterally inserted into an intervertebral space S' defined between an upper vertebra V1' and a lower vertebra V2'. In this embodiment, the upper and lower vertebrae V1', V2' are substantially parallel to one another, and as such, the prosthetic device 70 includes a pair of transverse plates 72, 74, which are substantially parallel to one another and are substantially perpendicular to an associated sagittal plate 76.

To accommodate insertion of the prosthetic device 70, the vertebral bodies V1', V2' can be prepared to accept the prosthetic device therebetween from the lateral approach by laterally forming elongate openings or slots 78, 80 in the vertebral endplates of the upper and lower vertebrae V1', V2', respectively, at a predetermined width and to a predetermined depth. The slots 78, 80 can be substantially aligned with each other to accommodate the sagittal plate 76. In one embodiment, the elongate slots 78, 80 are rectangular-shaped and are formed by chiseling or curetting. However, other methods of forming the slots 78, 80 are also contemplated as would occur to one of ordinary skill in the art, such as, for example, by drilling or reaming. Furthermore, as described above with respect to the prosthetic device 20, the prosthetic device 70 may be configured to form their own corresponding slots by engagement and impaction with the vertebrae V1, V2, and thus no preformed slots are necessary.

The present disclosure has been described relative to several preferred embodiments. For example, although described with respect to circumventing vascular structure 12, it is understood that the above-described prosthetic device 20 may be desirable for use in scenarios where vascular structure 12 is not present. Moreover, although described with respect to longitudinal and lateral insertion, it is understood that the prosthetic device 20 may be inserted into the intervertebral space S from a variety of other approaches such as the transforaminal approach. It is also understood that all spatial references, such as "longitudinal", "lateral", and "transverse", are for illustrative purposes only.

## Claims

1. A fusion-promoting prosthetic device (20) for insertion into an intervertebral space defined between first and second vertebral bodies (V1, V2) and for promoting fusion in the intervertebral space, comprising a sagitally-extending plate (22) having caudal and cephalad edges (34, 36), the caudal edge (34) being adapted for complete insertion within a first vertebral body ( V2) and the cephalad edge (36) being adapted for complete insertion within a second vertebral body (V1) adjacent to the first vertebral body (V2) **characterised by**, a first transverse plate (26) connected to the sagitally-extending plate (22), and a second transverse plate (24) connected to the sagitally-extending plate (22), the first and second transverse plates (24, 26) being adapted for complete insertion within the intervertebral space.

2. The prosthetic device (20) of claim 1 wherein the first transverse plate (26) comprises a bearing surface (44), the bearing surface (44) being adapted to engage the first vertebral body (V2).

3. The prosthetic device (20) of claim 1 wherein the second transverse plate (24) comprises a bearing surface (42), the bearing surface (42) being adapted to engage the second vertebral body (V1).

4. The prosthetic device (20) of claim 1 wherein the sagitally-extending plate (22) further comprises a plurality of openings (40) extending therethrough.

5. The prosthetic device (20) of claim 1 wherein each of the first and second transverse plates (24, 26) comprises a plurality of openings extending therethrough.

6. The prosthetic device (20) of claim 1 wherein the sagitally-extending plate (22) is coated with a bone-growth promoting substance.

7. The prosthetic device (20) of claim 1 wherein the first and second transverse plates (24, 26) are each coated with a bone-growth promoting substance.

8. The prosthetic device (20) of claim 1 wherein the first transverse plate (26) is angled relative to the sagitally-extending plate (22).

9. The prosthetic device (20) of claim 8 wherein the second transverse plate (24) is angled relative to the sagitally-extending plate (22), the angulation of the first and second transverse plates (24, 26) corresponding to an angular relationship defined between the first and second vertebral bodies (V1, V2).

10. The prosthetic device (20) of claim 1 wherein the first and second transverse plates (24, 26) each extend at a substantially right angle to the sagitally-extending plate (22).

11. The prosthetic device (20) of claim 1 wherein the caudal (34) edge is bevelled at a posterior portion thereof.

12. The prosthetic device of claim 1 wherein the cephalad edge (36) is bevelled at a posterior portion thereof.

## Patentansprüche

1. Eine fusionsfördernde Prothesenvorrichtung (20) zum Einsetzen in einen zwischen einem ersten und einem zweiten Wirbelkörper (V1, V2) definierten intervertebralen Raum und zum Fördern der Fusion in dem intervertebralen Raum, aufweisend eine sich sagittal erstreckende Platte (22), die einen kaudalen und einen kephalen Rand (34, 36) aufweist, wobei der kaudale Rand (34) für ein vollständiges Einsetzen in einen ersten Wirbelkörper (V2) angepasst ist und der kephale Rand (36) für ein vollständiges Einsetzen in einen zweiten Wirbelkörper (V1) benachbart zu dem ersten Wirbelkörper (V2) angepasst ist, **gekennzeichnet durch** eine erste Transversalplatte (26), die mit der sich sagittal erstreckenden Platte (22) verbunden ist, und eine zweite Transversalplatte (24), die mit der sich sagittal erstreckenden Platte (22) verbunden ist, wobei die erste und die zweite Transversalplatte (24, 26) für ein vollständiges Einsetzen in den intervertebralen Raum angepasst sind.

2. Die Prothesenvorrichtung (20) gemäß Anspruch 1, wobei die erste Transversalplatte (26) eine Lagerfläche (44) aufweist, wobei die Lagerfläche (44) für einen Eingriff mit dem ersten Wirbelkörper (V2) angepasst ist.

3. Die Prothesenvorrichtung (20) gemäß Anspruch 1, wobei die zweite Transversalplatte (24) eine Lagerfläche (42) aufweist, wobei die Lagerfläche (42) für einen Eingriff mit dem zweiten Wirbelkörper (V1) angepasst ist.

4. Die Prothesenvorrichtung (20) gemäß Anspruch 1, wobei die sich sagittal erstreckende Platte (22) ferner eine Mehrzahl von Öffnungen (40) aufweist, die sich durch diese hindurcherstrecken.

5. Die Prothesenvorrichtung (20) gemäß Anspruch 1, wobei jede von der ersten und der zweiten Transversalplatte (24, 26) eine Mehrzahl von Öffnungen aufweist, die sich durch diese hindurcherstrecken.

6. Die Prothesenvorrichtung (20) gemäß Anspruch 1, wobei die sich sagittal erstreckende Platte (22) mit einer knochenwachstumsfördernden Substanz beschichtet ist.

7. Die Prothesenvorrichtung (20) gemäß Anspruch 1, wobei die erste und die zweite Transversalplatte (24, 26) jeweils mit einer knochenwachstumsfördernden Substanz beschichtet sind.

8. Die Prothesenvorrichtung (20) gemäß Anspruch 1, wobei die erste Transversalplatte (26) bezüglich der sich sagittal erstreckenden Platte (22) abgewinkelt ist.

9. Die Prothesenvorrichtung (20) gemäß Anspruch 8, wobei die zweite Transversalplatte (24) bezüglich der sich sagittal erstreckenden Platte (22) abgewinkelt ist, wobei die Abwinkelung der ersten und der zweiten Transversalplatte (24, 26) mit einem zwischen dem ersten und dem zweiten Wirbelkörper (V1, V2) definierten Winkelverhältnis korrespondiert.

10. Die Prothesenvorrichtung (20) gemäß Anspruch 1, wobei die erste und die zweite Transversalplatte (24, 26) sich jeweils in einem im Wesentlichen rechten Winkel zu der sich sagittal erstreckenden Platte (22) erstrecken.

11. Die Prothesenvorrichtung (20) gemäß Anspruch 1, wobei der kaudale Rand (34) an einem posterioren Abschnitt davon abgefast ist.

12. Die Prothesenvorrichtung gemäß Anspruch 1, wobei der kephale Rand (36) an einem posterioren Abschnitt davon abgefast ist.

## Revendications

1. Dispositif prothétique (20) favorisant la fusion pour insertion dans un espace intervertébral défini entre les premier et second corps vertébraux (V1, V2) et pour favoriser la fusion dans l'espace intervertébral, comprenant une plaque d'extension sagittale (22) ayant des bords caudal et céphalique (34, 36), le bord caudal (34) étant adapté pour une insertion complète dans un premier corps vertébral (V2) et le bord céphalique (36) étant adapté pour une insertion complète dans un second corps vertébral (V1) adjacent au premier corps vertébral (V2), **caractérisé par** une première plaque transversale (26) raccordée à la plaque d'extension sagittale (22), et une seconde plaque transversale (24) raccordée à la plaque d'extension sagittale (22), les première et seconde plaques transversales (24, 26) étant adaptées pour une insertion complète dans l'espace intervertébral.

2. Dispositif prothétique (20) selon la revendication 1, dans lequel la première plaque transversale (26) comprend une surface de support (44), la surface de support (44) étant adaptée pour s'engager sur le premier corps vertébral (V2).

3. Dispositif prothétique (20) selon la revendication 1, dans lequel la seconde plaque transversale (24) comprend une surface de support (42), la surface de support (42) étant adaptée pour s'engager sur le second corps vertébral (V1).

4. Dispositif prothétique (20) selon la revendication 1, dans lequel la plaque d'extension sagittale (22) comprend en outre une pluralité d'ouvertures (40) qui la traversent.

5. Dispositif prothétique (20) selon la revendication 1, dans lequel chacune des première et seconde plaques transversales (24, 26) comprend une pluralité d'ouvertures qui la traversent.

6. Dispositif prothétique (20) selon la revendication 1, dans lequel la plaque d'extension sagittale (22) est revêtue d'une substance favorisant la croissance osseuse.

7. Dispositif prothétique (20) selon la revendication 1, dans lequel les première et seconde plaques transversales (24, 26) sont chacune revêtues d'une substance favorisant la croissance osseuse.

8. Dispositif prothétique (20) selon la revendication 1, dans lequel la première plaque transversale (26) fait un angle avec la plaque d'extension sagittale (22).

9. Dispositif prothétique (20) selon la revendication 8, dans lequel la seconde plaque transversale (24) fait un angle avec la plaque d'extension sagittale (22), l'angulation des première et seconde plaques transversales (24, 26) correspondant à une relation angulaire définie entre les premier et second corps vertébraux (V1, V2).

10. Dispositif prothétique (20) selon la revendication 1, dans lequel les première et secondes plaques transversales (24, 26) s'étend chacune en faisant un angle sensiblement droit avec la plaque d'extension sagittale (22).

11. Dispositif prothétique (20) selon la revendication 1, dans lequel le bord caudal (34) est biseauté dans sa partie postérieure.

12. Dispositif prothétique selon la revendication 1, dans lequel le bord céphalique (36) est biseauté dans sa partie postérieure.
